# EUROPEAN PATENT SPECIFICATION

(11) **EP 2 720 537 B1**
(45) Date of publication and mention of the grant of the patent: **14.02.2018**
(21) Application number: 12766817.6
(22) Date of filing: 19.09.2012
(51) Int. Cl.: A01N 1/02, A61J 1/10, B32B 27/08

(54) **RED BLOOD CELLS PRODUCTS AND THE STORAGE OF RED BLOOD CELLS IN NON-PVC BAGS**
PRODUKTE ENTHALTEND ROTE BLUTZELLEN UND LAGERUNG VON ROTEN BLUTZELLEN IN NICHT PVC-HALTIGEN BEUTELN
PRODUITS CONTENANT DES ÉRYTHROCYTES ET STOCKAGE DES ÉRYTHROCYTES DANS DES SACS SANS PVC

(30) Priority: 19.09.2011 US 201161536370 P; 20.10.2011 US 201161549562 P
(43) Date of publication of application: 23.04.2014
(73) Proprietor: Fenwal, Inc., Lake Zurich, IL 60047 (US)
(72) Inventor: MIN, Kyungyoon, Kildeer, IL 60047 (US); RADWANSKI, Katherine, Des Plaines, IL 60016 (US); SANDFORD, Craig, L., Buffalo Grove, IL 60089 (US)
(74) Representative: Prins, Hendrik Willem
(86) International application number: PCT/US2012/056011
(87) International publication number: WO 2013/043658

(56) References cited:
- DE-A1- 3 200 264
- US-A- 4 140 162
- US-A- 4 943 287
- US-A- 5 769 839
- US-A1- 2011 117 647
- H. R. HILL ET AL: "The effects of polyvinyl chloride and polyolefin blood bags on red blood cells stored in a new additive solution", VOX SANGUINIS, vol. 81, no. 3, 1 October 2001 (2001-10-01), pages 161-166, XP55045602, ISSN: 0042-9007, DOI: 10.1046/j.1423-0410.2001.00097.x
- C.J. DRAPER ET AL: "Biochemical and structural changes in RBCs stored with different plasticizers: the role of hexanol", TRANSFUSION, vol. 42, no. 7, 1 July 2002 (2002-07-01), pages 830-835, XP55045604, ISSN: 0041-1132, DOI: 10.1046/j.1537-2995.2002.00138.x
- HESS J R ET AL: "Successful storage of RBCs for 9 weeks in a new additive solution", TRANSFUSION, AMERICAN ASSOCIATION OF BLOOD BANKS, BETHESDA, MD, US, vol. 40, no. 8, 1 August 2000 (2000-08-01) , pages 1007-1011, XP007911856, ISSN: 0041-1132, DOI: 10.1046/J.1537-2995.2000.40081007.X

## Description

### BACKGROUND

Red blood cells are often separated from whole blood and collected for later transfusion to a patient in need of red blood cells. For example, red blood cells (hereinafter "RBCs") may be administered to a patient suffering from a loss of blood due to trauma, as a post-chemotherapy treatment, or as part of a treatment of one or more blood borne diseases, such as certain anemias and the like. Unless administered immediately after collection from a donor, RBCs must typically be stored for some period of time prior to transfusion. The storage period may be anywhere from a few days to several weeks.

Prolonged storage of RBCs can (negatively) affect RBC function. In order for the RBCs to be suitable for transfusion to the recipient, RBCs must maintain adequate cell function and metabolism. For example, RBCs must maintain an adequate concentration of adenosine triphosphate (ATP) and 2,3-DPG. In addition, the presence of lactate must not be too high in the stored RBCs. Still further, stored RBCs must have acceptably low levels of hemolysis. Typically, an acceptable level of hemolysis is below 1.0% (in, for example, the U.S.) and 0.8% (in Europe) after 42 day storage.

Media for providing a storage environment for RBCs that will allow cell function and cell metabolism to be preserved and maintained have been developed and are commonly used. The media developed for RBCs can prolong the storage life of RBCs for up to 42 days. Such media (or "storage solutions") often include a nutrient for the RBCs, a buffer to help maintain the pH of the RBCs, electrolytes, a RBC membrane-protecting compound and other additives to enhance and extend the life of the RBCs. Examples of widely used and accepted storage media are Adsol and SAG-M, available from Fenwal, Inc., of Lake Zurich, Illinois. Adsol and SAG-M include sodium chloride, glucose, mannitol, and adenine. Both Adsol and SAG-M have a pH of about 5.0 (referred to herein as "low pH") and are substantially isotonic.

Other additive solutions are disclosed in U.S. Patent Application Publication No. 2009/0239208 and 2011/0117647. The additive solutions disclosed therein are, hypotonic, synthetic aqueous storage solutions for the prolonged storage of RBCs. The storage media disclosed therein typically include adenine, mannitol, sodium citrate, sodium phosphate, and glucose as the nutrient. These hypotonic aqueous additive solutions have a "high" pH of at least about 8.0.

During storage, concentrated RBCs and the additive solutions in which they are stored are typically kept in a sealed container, usually made of a plastic material. Most typically, the containers approved for the collection of whole blood and the storage of RBCs are made of a polyvinyl chloride (PVC). Inasmuch as polyvinyl chloride can be somewhat rigid or brittle, a plasticizer is typically incorporated into the PVC. Examples of currently known and used plasticizers for medical grade PVC are DEHP, TEHTM, and the family of citrate esters described in U.S. Patent No. 5,026,347.

H.R. Hill et al.: "The effects of polyvinyl chloride and polyolefin blood bags on red blood cells stored in a new additive solution", VOX SANGUINIS, vol. 81, no. 3, 1 October 2001, pages 161-166, XP055045602, investigates the effects of PVC and polyolefin blood bags on red blood cells stored in an experimental additive solution (EAS 61).

J.R. Hess et al.: "Successful storage of RBCs for 9 weeks in a new additive solution", TRANSFUSION, American Association of Blood Banks, Bethesda, MD, US, vol. 40, no. 8, 1 August 2000, page 1007-1011, XP007911856 describes a study in which the use of EAS 61 in storing packed RBC's is explored. A pyrogen-free, sterile EAS 61 is disclosed having a pH of 8.23 at 25°C.

As reported in U.S. Patent No. 5,026,347 and other literature, such as Rock, et al. "Incorporation of plasticizer into red cells during storage," Transfusion, 1984; Horowitz et al. "Stablization of RBCs by the Plasticizer, Di(ethylhexyl)phthalate," Vox Sarguinis, 1985, plasticizers may also have a beneficial effect on the storage life of RBCs. More particularly, plasticizers such as DEHP and the family of citrate esters have been found to suppress hemolysis of RBCs stored in containers that include such leachable plasticizers. RBCs stored in containers made of plasticized PVC or a non-PVC container with plasticizer added (as described in U.S. Patent No. 5,026,347) have traditionally been combined with an isotonic, low pH storage solution (such as Adsol). While DEHP plasticized PVC containers have worked well for the storage of red blood cells, the use of other container materials and additive solutions to provide a suitable storage environment for the red blood cells remains a topic of keen interest.

Thus, it would be desirable to provide a storage environment for RBCs wherein the container is made of a non-PVC material and is at least substantially free of any leachable phthalate plasticizer and the storage media is a hypotonic and high pH solution. In addition, it would be desirable to provide a storage environment for RBCs, wherein the container is made of a non-PVC material that is at least substantially free of any plasticizer and the storage media is a hypotonic and high pH solution. As used herein, the term "storage environment" refers to the materials and solutions that contact the RBCs during storage.

### SUMMARY

The present invention relates to the red blood cell product of independent Claim 1 and the method for providing a transfusible red blood cell product of independent Claim 8. The dependent claims recite preferred but optional features.

In one aspect, the present disclosure is directed to RBC products. The products include a container in which the wall of the container defines an interior chamber. At least a portion of the wall is made of non-PVC, substantially plasticizer-free material. According to the present invention, the wall is made of a non-PVC plasticizer-free plastic, wherein said non-PVC, plasticizer-free plastic comprises a polypropylene homopolymer, co-polymer or blends thereof, or ethylene vinylacetate. The product further includes a suspension of RBCs contained within the chamber. The suspension includes concentrated RBCs in a hypotonic solution. The hypotonic, chloride-free solution includes at least a nutrient, a buffer, and has a pH of greater than approximately 8.0.

In another aspect, the present disclosure is directed to a transfusible RBC composition. The composition includes a suspension of RBCs that includes concentrated RBCs substantially free of plasticizer and having a hemolysis level of below at least 1.0% for its storage life.

In a further aspect, the present disclosure is directed to a RBC product that includes a plastic container made of a material consisting essentially of a plasticizer-free, non-PVC material. The product includes a RBC suspension that includes concentrated RBCs and a hypotonic solution that includes at least a nutrient, a buffer, and has a pH of at least about 8.0.

In yet another aspect, the present disclosure is directed to a method for providing a transfusible RBC product that is substantially free of plasticizer. The product includes deriving a RBC concentrate from whole blood and combining the RBC concentrate with a hypotonic solution that includes at least one nutrient, a buffer, and has a pH of at least 8.0 or higher. The method further includes storing the combination of RBC concentrate and hypotonic solution in a container made of a non-PVC, substantially plasticizer-free material. According to the method of the present invention, the combination of RBC and hypotonic solution is stored in a container that is made of a non-PVC plasticizer-free plastic, wherein said non-PVC, plasticizer-free plastic comprises a polypropylene homopolymer, co-polymer or blends thereof, or ethylene vinylacetate.

### BRIEF DESCRIPTION OF THE DRAWINGS

Figure 1 is a plan view of a typical RBC storage container used for storing the RBC suspension described herein;
Figure 2 is a side view of the container of Fig. 1;
Figure 3 is a graph comparing the levels of hemolysis in various containers having different amounts of a plasticizer (including no plasticizer);
Figure 4 is a graph showing the levels of ATP over a 42-day storage period of RBCs stored in containers having varying levels of plasticizer (including no plasticizer);
Figure 5 is a graph showing the levels of 2,3-DPG over a 42-day storage period of RBCs stored in containers having varying levels of plasticizer (including no plasticizer); and
Figure 6 is a graph showing the levels of lactate over a 42-day storage period of RBCs stored in containers having varying degrees of plasticizer (including no plasticizer).

### DETAILED DESCRIPTION

Disclosed herein are RBC products. RBC products, as disclosed herein, include (a) a RBC composition, and (b) a container for holding the composition during a period of storage. The RBC composition itself includes concentrated RBCs that have been combined with an additive solution selected to maintain cell function and metabolism of the RBCs during prolonged storage (e.g., at least about 42 days and possibly even up to at least 49 and/or 56 days). The container typically (but not exclusively) is made of a plastic material and more specifically a plastic material that does not include polyvinylchloride (PVC), a phthalate plasticizer, or any plasticizer whatsoever. The RBCs of the RBC product are suitable for transfusion to a patient.

As noted above, RBC compositions include RBC concentrate and an additive solution. Concentrated RBCs are derived from whole blood either by manual or automated separation collection techniques which will be known to those skilled in the art. RBC concentrates may include some residual amount of plasma. In one embodiment, the RBC concentrate may have most of its plasma removed as described, for example, in International Application Publication WO/2011/049709.

Regardless of how much plasma remains with the RBCs, the RBC compositions of the present disclosure also include an additive solution. In one embodiment, the additive solution is one that allows for the extended storage of RBCs (in the containers described herein) for over 21 days, over 35 days, up to at least 42 days, and even up to at least 49 and/or 56 days. Additive solutions suitable for the storage of RBCs in accordance with the present disclosure are generally hypotonic and typically (but not necessarily) do not include sodium chloride. Such storage solutions also include a nutrient, a buffer and other additives such as sodium citrate. Solutions suitable for use in the storage of RBCs in accordance with the present disclosure typically have a pH of about 8.0 or higher. Examples of additive solutions are described in U.S. Patent Publication Nos. US 2009/0239208 and US 2011/0117647. In a specific embodiment, the additive solutions include between about 1 to 2.2 mM of adenine; about 20 mM to about 110 mM of mannitol; about 2.2 mM to about 40 mM sodium citrate; about 16 mM to about 30 mM sodium phosphate dibasic and about 20 mM to about 140 mM of glucose. The pH of the additive solution is above about 8.0.

In a more specific example, the additive solution useful in the storage of concentrated RBCs in accordance with the present disclosure includes about 2.0 mM of adenine; about 41 mM of mannitol; or about 25 mM of sodium citrate; about 20 mM of sodium phosphate dibasic and about 111 mM of glucose. The solution described above is referred to herein as "E-Sol 5."

Thus, concentrated RBCs with some or most of the plasma removed are combined with additive solutions of the type described above to provide the RBC composition. In one embodiment, the RBC composition includes between about 80 to 150 ml of the additive solution combined with about 180 to 250 ml of the concentrated RBCs. More preferably, the volume of additive solution may be about 100-110 ml.

In the collection of RBCs, it is typical to remove leukocytes from, or at least reduce the number of leukocytes in, the RBCs prior to their storage and transfusion. RBCs suspended in an additive solution are often subjected to a leuko-reduction step which commonly includes filtration of the RBC/additive solution.

In accordance with the methods and systems disclosed herein, RBCs are subjected to a filtration step or other treatment whereby one or both of leukocytes and prions are substantially removed (or the populations of leukocytes and/or prions are substantially reduced) from the RBCs. In one embodiment, concentrated RBCs may be combined with an additive solution of the type described above and the combined concentrated RBC/additive solution composition may be subjected to the leukocyte and/or prion removal (e.g., filtration) step.

In another embodiment, the RBC concentrate may be "leuko-reduced" and/or "prion-reduced" prior to combination with the additive solution. Thus, in this embodiment, RBC concentrate separated from whole blood is filtered by passing the RBC concentrate through a leuko-reduction filter. Alternatively, the whole blood may be subjected to leuko-reduction (i.e., leuko-filtration) and/or prion reduction/removal prior to separation of the RBCs from the whole blood. In any event, the RBCs are "leuko-reduced and/or "prion-reduced." Filters suitable for removing leukocytes (and/or prions) from whole blood or RBC concentrate (prior to the addition of the additive solution) include, but are not limited to, the Sepacell R-500 II, RZ-2000, RS-2000, Flex-Excel, Pure-RC, RZ-200 and R-3000. (Of course, other means for removing and/or reducing the number of leukocytes may also be used.) In this embodiment, the (now) leuko-reduced RBC concentrate is combined with the hypotonic additive solution of the type described above. The hypotonic additive solution may be added after introduction of the RBC concentrate into the container, or may already be present in the container at the time of RBC concentrate introduction.

Leuko-reduced (and/or prion-reduced) RBC concentrate (either with or without additive solution) is then introduced into a container which may be made of a non-PVC material that is at least substantially free of any plasticizer.

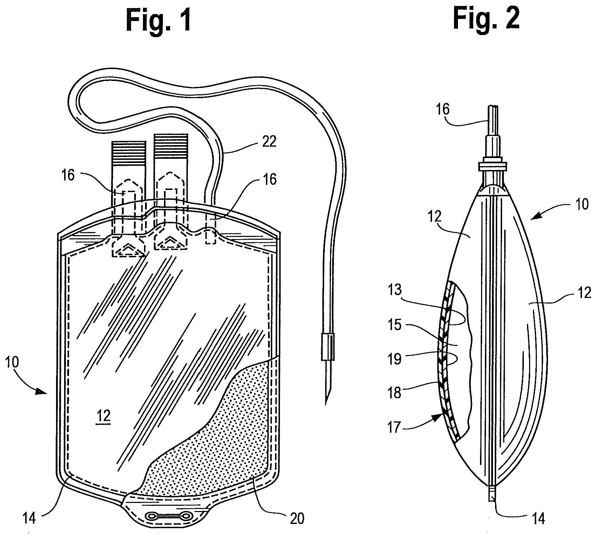

The compositions described above may be provided in a container that is suitable for the long term storage of RBCs. Preferably, containers for storing the RBC compositions disclosed herein are made of a non-PVC (non-polyvinyl chloride) plastic material. The containers may be permeable to oxygen or at least semi-permeable to oxygen. As shown in Figs. 1 and 2, container 10 may include one or more container walls 12 which define an interior chamber 15 for receiving the RBC composition 20. In one embodiment, two sheets made of a plastic material are brought together and sealed along their peripheries 14 to form container 10. Other ways of making container 10 will be known to those of skill in the art and are within the scope of the present disclosure. As shown in Fig. 2, container wall 12 includes an inner surface 13 which contacts the RBCs and an outer surface 17. In one embodiment, container wall 12 may be made of a multiple sheet laminate wherein inner surface 13 is made of one material and outer surface 17 is made of a different material. Container 10 may include one or more access ports 16 for connection with tubing 22, docking devices and the like to establish flow into and out from the interior chamber 15 of container 10.

In one embodiment, containers useful in the storage of RBCs as described above include container walls that are made in whole or at least in part of a non-PVC plastic material that may include at least one or more polymeric compounds. The one or more plastic and/or polymeric compounds may be blended together and formed into flat sheets that are sealed together in the manner described above. In one embodiment, the polymeric material may be made from or otherwise include one or more polyolefin homopolymers, co-polymers or blends thereof. Examples of suitable polyolefins include polypropylene, polyethylene, including ultra low density polyethylene (ULDPE) and very low density polyethylene (VLDPE). Other suitable compounds that may be used in the plastic materials of the containers or as part of the blend for making the plastic materials include ethylene vinylacetate (EVA) and block co-polymers such as Kraton. Exemplary formulations and/or the polyolefins, polyolefin blends or other polymeric compounds which are useful, either alone or in combination, in the manufacture of containers suitable for use in the RBC products of the present disclosure are described in U.S. Patent No. 5,026,347, 4,140,162, 5,849,843, and 6,579,583.

As indicated above, the structure of the container or container walls may include one, two or more layers. The layer formulations may include one, two, three or more components. These structures should be suitable for sterilization by appropriate means, such as steam, ionizing radiation or ethylene oxide. Structures suitable for steam sterilization should resist distortions to high temperatures up to 121°C. This typically requires incorporation of materials with a melting peak of greater than 130°C. The preferred structure of autoclavable material suitable for the invention will incorporate polypropylene homopolymer or copolymer at a level of 30% or more in at least one of the layers to provide thermal resistance. A suitable polypropylene copolymer is supplied by Total Petrochemicals (random copolymer 6575). However, thermal resistance can also be obtained by crosslinking a lower melting material. For example, a 28% vinyl acetate EVA can be crosslinked by ionizing radiation sufficiently to withstand autoclave temperatures even though it has a melting point of 76°C. Suitable materials include Arkema Evatane® 28-03 and Celanese Ateva® 2803. The preferred structure is highly flexible, having a composite modulus of not more than 20,000 psi.

In some cases, it may be desirable for the container walls to have radio frequency (RF) response to enable heat sealing. This can be accomplished by incorporating an RF responsive material such as described in U.S Patent 5,849,843.

Preferred structures for radiation sterilized applications will incorporate at least 30% of an ethylene based polymer (LDPE, LLDPE, ULDPE, VLDPE, EVA) in at least one of the layers. Structures of polypropylene copolymers and polypropylene polymers blended with elastomers such as Kraton® or ULDPE are also suitable for radiation sterilized applications. The preferred structure incorporates lower modulus components in at least one of the layers to enhance flexibility and toughness. These lower modulus components can be ultralow density polyethylene (ULDPE - typical density less than 0.90 Kg/L), very low density polyethylene (VLDPE - typical density less than 0.925 Kg/L), ethylene vinyl acetate copolymers (EVA) with greater than 16% vinyl acetate content, styrene butadiene terpolymers such as Kraton® ULDPE materials are commercially available as Mitsui TAFMER®, Exxon Mobil Exact® and Dow Affinity®. EVA materials are available as Arkema Evatane® and Celanese Ateva®. These materials are incorporated at levels sufficient to obtain a composite modulus of less than 20,000 psi while maintaining resistance to distortion at temperatures greater than 121°C for autoclaved applications. The disclosure of suitable non-PVC plastics set forth above is not meant to be exhaustive and that other non-PVC plastics, polymers and blends thereof may also be used in the products and compositions of the present disclosure.

The formulations used to make container walls 12 of container 10 are at least substantially free of polyvinylchloride (PVC). At the very least, surface 13 of container wall 12 is substantially free of PVC. In an embodiment where container 10 is made of a multiple sheet laminate the sheet providing inner surface 13 may be made substantially of a non-PVC material while the sheet providing outer surface 17 may be made of a different material. Alternatively, the container wall 12 may be made of a single sheet of a non-PVC polyolefin or other plastic, as described above.

In addition to being substantially PVC free (i.e., non-PVC), containers suitable for use in the products, systems and methods of the present disclosure are at least substantially free of phthalate plasticizer or at least substantially free of any plasticizer whatsoever. More particularly, at least a portion of the container wall, i.e., the portion or surface that is in contact with the RBCs during storage, is at least substantially free of phthalate plasticizer or at least substantially free of any plasticizer. For example, at least inner surface 13 (or that portion of inner surface 13 that is in contact with the RBCs) may be substantially free of PVC and substantially free of phthalate plasticizer or any plasticizer at all. Thus, the storage environment in which the RBCs reside is at least substantially PVC-free, at least substantially free of phthalate plasticizer or any plasticizer and includes a hypotonic solution.

For example, in the embodiment where the non-PVC container (or more specifically, the container wall) is at least substantially free of a phthalate plasticizer, such a container may include a non-phthalate plasticizer such as triethylhexyltrimellitate (TEHTM), one or more of the citrate esters described in U.S. Patent No. 5,026,347, or the plasticizer known to those of skill in the art by its acronym DINCH. Accordingly, such non-phthalate plasticizer(s) will be present in and part of the RBC storage environment.

Where the container walls (or at least the inner surface(s) 13 of the walls) are made of a material completely free of plasticizer, some small trace amounts of plasticizer may be present in the container walls as a result of migration from adjoining or adjacent containers, from PVC tubing and/or the surrounding environment generally. In addition, as described above, ports 16 may likewise include PVC and as a result may include some plasticizer (including DEHP). Nonetheless, the presence of some trace amounts of plasticizer attributable to migration from other containers or tubing, or present in the plastic ports 16, is negligible and such containers are referred to herein as "substantially plasticizer-free" or "substantially free of plasticizer."

The compositions of the present disclosure may also include other additives such as anti-blocking and slip agents. Examples of such anti-blocking and slip agents include derivatives of fatty acid and ethylenediamine. More specifically, the agents may be longer chain fatty acids, containing 12 or longer hydrocarbon chains with or without mono-unstaurated carbon-carbon bonds, based daiamide with ethylendiamine, such as n,n'-ethylene bissteararamide and n,n'-dioleoyl ethylenediamine. Commercially available compounds of the type described above and which may be used in the non-PVC, non-plasticized compositions of the present disclosure include Acrawax and Glycolube, both available from Lonza of Basel, Switzerland. The anti-blocking and/or slip agents may be coated onto the interior surface of the containers or otherwise incorporated therein.

RBC compositions (which include RBC concentrates and additive solutions) may be stored in the containers in the substantially PVC free and substantially phthalate-free or substantially plasticizer-free containers described above. The RBC compositions stored in such containers may be stored for more than 21 days, more than 35 days and up to at least 42 days or even up to at least 49 days and/or 56 days, while maintaining acceptable storage cell function parameters (i.e., a level of ATP, 2,3-DPG, lactate). In particular, RBC compositions stored in the polyolefin-based containers that are substantially PVC-free and substantially plasticizer-free that include a hypotonic solution of the type described above maintain hemolysis levels below 1.0% and even below 0.8% at, for example, 42 days of storage. Similarly, the RBC compositions stored for at least about 42 days also include ATP, 2,3-DPG, lactate, potassium, phosphate levels that are comparable to RBC compositions stored in plasticized PVC containers, as shown in the study described below.

### Study 1

Whole blood units were collected in CPD anticoagulant in commercially available blood pack units. Within 15 minutes of collection, the units were transferred to non-PVC, non-DEHP polyolefin based containers for pooling. Three units of ABO matched whole blood were pooled together and split back into non-DEHP, non-PVC, polyolefin-based containers. The units were leukofiltered, centrifuged, and processed into plasma and concentrated red cells. Approximately 105 ml of E-Sol 5 was added to each RBC concentrate. The RBC concentrates were then transferred to (1) a container made of a standard PVC, plasticized with a DEHP-plasticizer (referenced in the Figures as 1.0 DEHP, PVC); (2) a container made of a non-PVC, oxygen semi-permeable material that is a steam sterilizable, multi-component blend that includes a copolymer of polypropylene as its major component with 50% less of the DEHP-plasticizer than the container in (1) above (referenced in the Figures as 0.5 DEHP, non-PVC A), and (3) a container made of a non-PVC-free, plasticizer-free material radiation sterilizable multi-component blend including primarily ethylene vinyl acetate (referenced in the Figures as 0.0 DEHP, non-PVC B).

Units were stored upright for 42 days at 4°C, with weekly sampling for in vitro parameters. On Day 42 DEHP content in the RBC concentrate was also measured after thorough mixing of each unit.

### Study 2

In a further study, whole blood units were collected into CPD anticoagulant in commercially available PVC, plasticizer blood pack units. Within 15 minutes of collection, whole blood units were leukofiltered, centrifuged, and processed into plasma and concentrated RBC component. Approximately 105 ml of E-Sol 5 was added to each red cell concentrate, which was transferred to a container made of a semi gas-permeable, non-PVC material as used in the container of subsection (2) of Study 1 described above but substantially free of any plasticizer (referenced in the Figures as 0.0 DEHP, non-PVC A).

Units were stored upright for 42 days at 4°C, with weekly sampling for in vitro parameters. On Day 42 of storage, DEHP content in the RBC concentrate was also measured after thorough mixing of each unit.

### Study 3

Whole blood units were collected in CPD anticoagulant in commercially available PVC, plasticizer blood pack units. Within 15 minutes of collection, the units were transferred to non-PVC, non-DEHP polyolefin based containers for pooling. Two units of ABO matched whole blood were pooled together and split back into the original collection containers. The units were leukofiltered, centrifuged, and processed into plasma and concentrated red cells. Approximately 110 ml of Adsol was added to each RBC concentrate which was then stored in a container identical to the container used to store red cell concentrate in E-Sol as described above in Study 2 (referenced in the Figures as 0.0 DEHP, non-PVC A, Adsol).

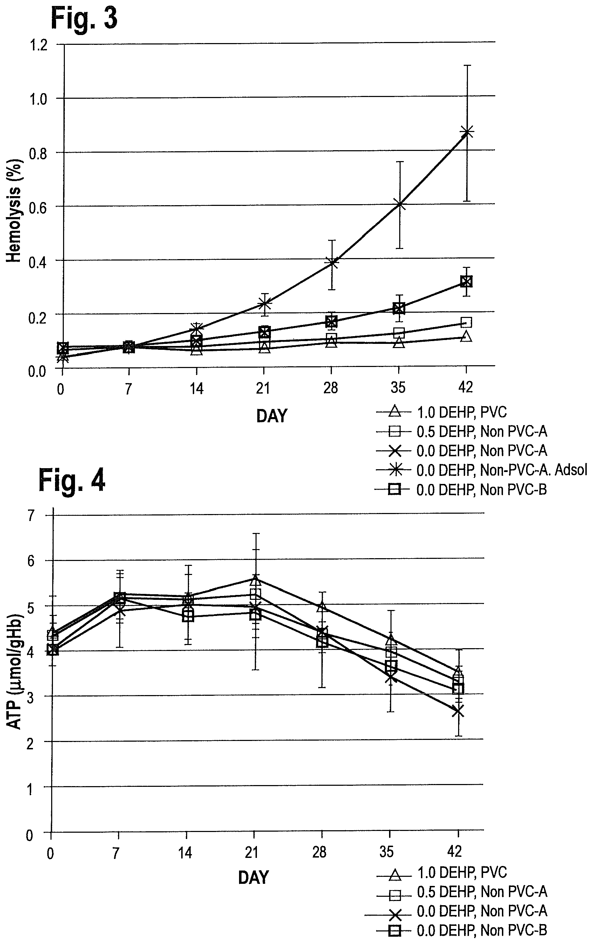

Paired units were stored upright for 42 days at 4°C, with weekly sampling for in vitro parameters in one unit from each pair (data shown in Fig 3) and Day 0 and Day 42 sampling only in the other unit from each pair (data not shown). On Day 42 of storage, DEHP content in the RBC concentrate was also measured after thorough mixing of each unit.

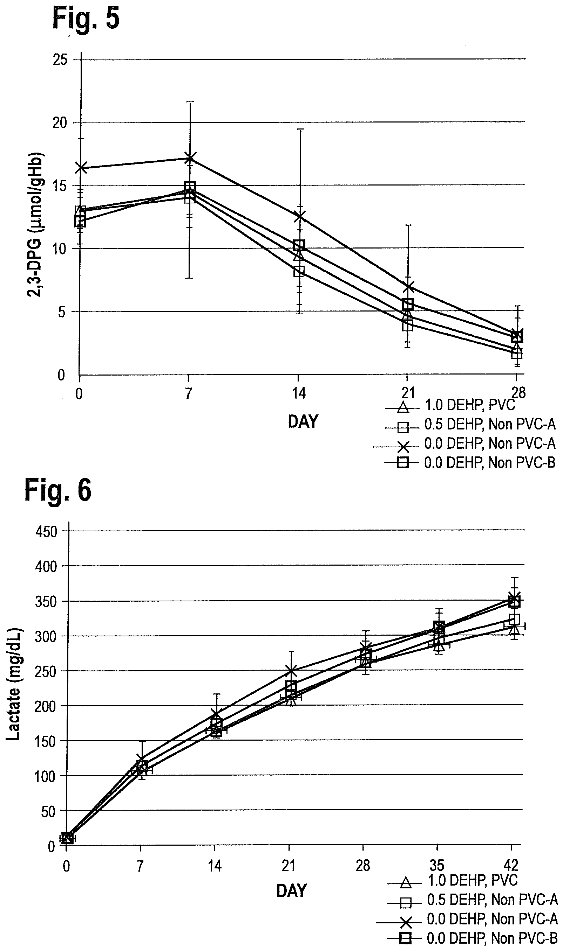

No major differences were observed in hematocrit, pH, glucose, lactate, phosphate, potassium, 2,3-DPG, and red cell micro particles among the three arms of Study 1 and the single arm of Study 2 described above. As shown in Fig. 3, all E-Sol 5 units passed accepted criteria for hemolysis (below 1.0% and 0.8%, respectively), at Day 42. The Adsol units described in Study 3 had 0.8% hemolysis on average on Day 42. ATP, 2,3-DPG and lactate levels were also comparable in the E-Sol stored units, as also shown in Figs. 4-6. With reference to Table 1 below, DEHP levels in the containers in the non-PVC, non-plasticized containers from Studies 1 and 2 showed negligible DEHP levels of 0.7 ± 0.2 and 2.3 ± 0.8, respectively in the RBC compositions, thereby indicating that the RBC compositions that are "substantially plasticizer-free" and stored in E-Sol 5 maintained acceptable hemolysis levels.

| Study | Container/Additive Solution | n | DEHP Content at Day 42 |
|---|---|---|---|
| 1 | 1.0 DEHP, PVC E-Sol 5 | 9 | 34.9 ± 4.7 |
| 1 | 0.5 DEHP, Non PVC-A E-Sol 5 | 8 | 16.7 ± 6.4 |
| 1 | 0.0 DEHP, Non-PVC-B E-Sol 5 | 10 | 0.7 ± 0.2 |
| 2 | 0.0 DEHP, Non PVC-A E-Sol 5 | 9 | 2.3 ± 0.8 |
| 3 | 0.0 DEHP, Non PVC-A Adsol | 6 | 1.8 ± 0.5 |

## Claims

1. A red blood cell product comprising:
(a) a container comprising a wall defining an interior chamber wherein the wall is made of a non-PVC, plasticizer-free plastic, wherein said non-PVC, plasticizer-free plastic comprises a polypropylene homopolymer, co-polymer or blends thereof, or ethylene vinylacetate;
(b) a suspension of red blood cells contained within said chamber, said suspension comprising:
(i) concentrated red blood cells; and
(ii) a hypotonic solution comprising at least a nutrient, a buffer and having a pH of at least 8.0;
wherein the hypotonic solution is a chloride-free solution comprising:
1 mM to 2.2 mM adenine;
20 mM to 110 mM mannitol;
2.2 mM to 90 mM sodium citrate;
16 mM to 30 mM sodium phosphate dibasic; and
20 mM to 140 mM glucose, wherein the pH of the aqueous storage solution is at least 8.0.

2. The red blood cell product of Claim 1 wherein said non-PVC, plasticizer-free material is a polyolefin.

3. The product of Claim 1 wherein said non-PVC, plasticizer-free material comprises a block co-polymer, ultra low density polyethylene and ethylene vinyl acetate.

4. The product of any of the claims 1-3 wherein said suspension comprises concentrated red blood cells substantially free of plasticizer and having a level of hemolysis that is below 1.0%.

5. The product of Claim 4, wherein said suspension comprises concentrated red blood cells substantially free of plasticizer and having a level of hemolysis that is below 0.8%.

6. The product of Claim 4 wherein the level of hemolysis is below 1.0% after 42 days of storage.

7. The product of Claim 6 wherein the level of hemolysis is below 0.8% after 42 days of storage in a non-PCV, non-plasticized plastic container.

8. A method for providing a transfusible red blood cell product substantially free of plasticizer comprising:
(a) deriving red blood cell concentrate from whole blood;
(b) combining said concentrated red blood cells with a hypotonic solution comprising at least a nutrient, a buffer and having a pH of at least 8.0; and
(c) storing said combination of concentrated red blood cells and a hypotonic solution in a container that is made of a non-PVC, plasticizer-free material, wherein
said non-PVC, plasticizer-free plastic comprises a polypropylene homopolymer, co-polymer or blends thereof, or ethylene vinylacetate;
wherein the hypotonic solution is a chloride-free solution comprising:
1 mM to 2.2 mM adenine;
20 mM to 110 mM mannitol;
2.2 mM to 90 mM sodium citrate;
16 mM to 30 mM sodium phosphate dibasic; and
20 mM to 140 mM glucose, wherein the pH of the aqueous storage solution is at least 8.0.

9. The method of Claim 8 wherein said red blood cell product is stored in said container for at least 42 days and the level of hemolysis of the concentrated red blood cells is below 1.0%.

10. The method of Claim 9, wherein said red blood cell product is stored in said container for at least 42 days and the level of hemolysis of the concentrated red blood cells is below 0.8%.

11. The method of Claim 8 or 9 wherein said non-PVC, plasticizer-free material is a polyolefin.

12. The method according to Claim 8 or 9 wherein said non-PVC, plasticizer-free material comprises a block co-polymer, ultra low density polyethylene and ethylene vinyl acetate.

## Patentansprüche

1. Produkt enthaltend rote Blutzellen, mit:
(a) einem Behälter mit einer Wand, die eine Innenkammer definiert, wobei die Wand aus einem nicht PVC-haltigen, weichmacherfreien Kunststoff besteht, wobei dieser nicht PVC-haltige, weichmacherfreie Kunststoff ein Polypropylen-Homopolymer, Copolymer oder Mischungen aus diesen oder Ethylen-Vinylacetat umfasst;
(b) einer in der Kammer enthaltenen Suspension aus roten Blutzellen, wobei die die Suspension Folgendes umfasst:
(i) konzentrierte rote Blutzellen; und
(ii) eine hypotonische Lösung mit mindestens einem Nährstoff, einem Puffer und mit einem pH von mindestens 8,0;
wobei die hypotonische Lösung aus einer chloridfreien Lösung besteht, mit:
1 mM bis 2,2 mM Adenin;
20 mM bis 110 mM Mannitol;
2,2 mM bis 90 mM Natriumcitrat;
16 mM bis 30 mM Natriumphosphat zweibasig; sowie
20 mM bis 140 mM Glukose, wobei der pH der wässrigen Lagerlösung bei mindestens 8,0 liegt.

2. Produkt enthaltend rote Blutzellen nach Anspruch 1, wobei das nicht PVC-haltige, weichmacherfreie Material aus einem Polyolefin besteht.

3. Produkt nach Anspruch 1, wobei das nicht PVC-haltige, weichmacherfreie Material ein Block-Copolymer, Polyethylen mit ultraniedriger Dichte sowie Ethylen-Vinylacetat umfasst.

4. Produkt nach einem der Ansprüche 1-3, wobei die Suspension konzentrierte rote Blutzellen umfasst, die im Wesentlichen frei von Weichmacher sind und einen Hämolyse-Spiegel aufweisen, der unter 1,0% liegt.

5. Produkt nach Anspruch 4, wobei die Suspension konzentrierte rote Blutzellen umfasst, die im Wesentlichen frei von Weichmacher sind und einen Hämolyse-Spiegel aufweisen, der unter 0,8% liegt.

6. Produkt nach Anspruch 4, wobei der Hämolyse-Spiegel nach 42 Tagen Lagerung unter 1,0% liegt.

7. Produkt nach Anspruch 6, wobei der Hämolyse-Spiegel nach 42 Tagen Lagerung in einem nicht PVC-haltigen, weichmacherfreien Kunststoffbehälter unter 0,8% liegt.

8. Verfahren zur Bereitstellung eines übertragbaren Produktes enthaltend rote Blutzellen, das im Wesentlichen frei von Weichmacher ist, mit den Schritten:
(a) Ableiten eines Konzentrates aus roten Blutzellen aus dem gesamten Blut;
(b) Kombinieren dieser konzentrierten roten Blutzellen mit einer hypotonischen Lösung, die mindestens einen Nährstoff, einen Puffer umfasst und einen pH von mindestens 8,0 aufweist; und
(c) Lagern der Kombination aus konzentrierten roten Blutzellen und einer hypotonischen Lösung in einem Behälter, der
aus einem nicht PVC-haltigen, weichmacherfreien Material besteht, wobei der nicht PVC-haltige, weichmacherfreie Kunststoff ein Polypropylen-Homopolymer, Copolymer oder Mischungen aus diesen oder Ethylen-Vinylacetat umfasst;
wobei die hypotonische Lösung aus einer chloridfreien Lösung besteht, mit:
1 mM bis 2,2 mM Adenin;
20 mM bis 110 mM Mannitol;
2,2 mM bis 90 mM Natriumcitrat;
16 mM bis 30 mM Natriumphosphat zweibasig; sowie
20 mM bis 140 mM Glukose, wobei der pH der wässrigen Lagerlösung bei mindestens 8,0 liegt.

9. Verfahren nach Anspruch 8, wobei das Produkt enthaltend rote Blutzellen in dem Behälter über mindestens 42 Tage gelagert wird und der Hämolyse-Spiegel der konzentrierten roten Blutzellen unter 1,0% liegt.

10. Verfahren nach Anspruch 9, wobei das Produkt enthaltend rote Blutzellen in dem Behälter über mindestens 42 Tage gelagert wird und der Hämolyse-Spiegel der konzentrierten roten Blutzellen unter 0,8% liegt.

11. Verfahren nach Anspruch 8 oder 9, wobei das nicht PVC-haltige, weichmacherfreie Material aus einem Polyolefin besteht.

12. Verfahren nach Anspruch 8 oder 9, wobei das nicht PVC-haltige, weichmacherfreie Material ein Block-Copolymer, Polyethylen von ultraniedriger Dichte sowie Ethylen-Vinylacetat umfasst.

## Revendications

1. Produit d'érythrocytes comprenant :
(a) un récipient comprenant une paroi définissant une chambre intérieure dans lequel la paroi est constituée d'un plastique différent du PVC et sans plastifiant, dans lequel ledit plastique différent du PVC et sans plastifiant comprend un homopolymère de polypropylène, un copolymère ou des mélanges de ceux-ci, ou de l'éthylène acétate de vinyle ;
(b) une suspension d'érythrocytes contenue à l'intérieur de ladite chambre, ladite suspension comprenant :
(i) des érythrocytes concentrés ; et
(ii) une solution hypotonique comprenant au moins un nutriment, un tampon et ayant un pH d'au moins 8,0 ;
dans lequel la solution hypotonique est une solution dépourvue de chlorure comprenant :
de 1 mM à 2,2 mM d' adénine ;
de 20 mM à 110 mM de mannitol ;
de 2,2 mM à 90 mM de citrate de sodium ;
de 16 mM à 30 mM de phosphate de sodium dibasique ; et
de 20 mM à 140 mM de glucose, dans lequel le pH de la solution de stockage aqueuse est d'au moins 8,0.

2. Produit d'érythrocytes selon la revendication 1, dans lequel ledit matériau différent du PVC et sans plastifiant est une polyoléfine.

3. Produit selon la revendication 1, dans lequel ledit matériau différent du PVC et sans plastifiant comprend un copolymère séquencé, du polyéthylène ultra-basse densité et de l'éthylène acétate de vinyle.

4. Produit selon l'une quelconque des revendications 1 à 3, dans lequel ladite suspension comprend des érythrocytes concentrés sensiblement sans plastifiant et ayant un niveau d'hémolyse qui est inférieur à 1,0 %.

5. Produit selon la revendication 4, dans lequel ladite suspension comprend des érythrocytes concentrés sensiblement dépourvus de plastifiant et ayant un niveau d'hémolyse qui est inférieur à 0,8 %.

6. Produit selon la revendication 4, dans lequel le niveau d'hémolyse est inférieur à 1,0 % après 42 jours de stockage.

7. Produit selon la revendication 6, dans lequel le niveau d'hémolyse est inférieur à 8,0 % après 42 jours de stockage dans un récipient en plastique différent du PVC et non plastifié.

8. Procédé de fourniture d'un produit d'érythrocytes pouvant être transfusé sensiblement dépourvu de plastifiant comprenant :
(a) l'obtention d'un concentré d'érythrocytes à partir de sang total ;
(b) la combinaison desdits érythrocytes concentrés avec une solution hypotonique comprenant au moins un nutriment, un tampon et ayant un pH d'au moins 8,0 ; et
(c) le stockage de ladite combinaison d'érythrocytes concentrés et d'une solution hypotonique dans un récipient qui est constitué d'un matériau différent du PVC et sans plastifiant, dans lequel
ledit plastique différent du PVC et sans plastifiant comprend un homopolymère de polypropylène, un copolymère ou des mélanges de ceux-ci, ou de l'éthylène acétate de vinyle ;
dans lequel la solution hypotonique est une solution sans chlorure comprenant :
de 1 mM à 2,2 mM d' adénine ;
de 20 mM à 110 mM de mannitol ;
de 2,2 mM à 90 mM de citrate de sodium ;
de 16 mM à 30 mM de phosphate de sodium dibasique ; et
de 20 mM à 140 mM de glucose, dans lequel le pH de la solution de stockage aqueuse est d'au moins 8,0.

9. Procédé selon la revendication 8, dans lequel ledit produit d'érythrocytes est stocké dans ledit récipient pendant au moins 42 jours et le niveau d'hémolyse des érythrocytes concentrés est inférieur à 1,0 %.

10. Procédé selon la revendication 9, dans lequel ledit produit d'érythrocytes est stocké dans ledit récipient pendant au moins 42 jours et le niveau d'hémolyse des érythrocytes concentrés est inférieur à 0,8 %.

11. Procédé selon la revendication 8 ou 9, dans lequel ledit matériau différent du PVC et sans plastifiant est une polyoléfine.

12. Procédé selon la revendication 8 ou 9, dans lequel ledit matériau différent du PVC et sans plastifiant comprend un copolymère séquencé, du polyéthylène ultra-basse densité et de l'éthylène acétate de vinyle.
